# EUROPEAN PATENT SPECIFICATION

(11) **EP 1 074 267 B1**
(45) Date of publication and mention of the grant of the patent: **15.03.2006**
(21) Application number: 00306263.5
(22) Date of filing: 24.07.2000
(51) Int. Cl.: A61K 39/39, A61K 48/00, A61P 37/04

(54) **Induction of antigen-specific T cells by interferon**
Induktion von antigen-spezifischen T-Zellen durch Interferon
Induction par l'interféron de cellules T spécifiques pour un antigène

(30) Priority: 22.07.1999 JP 20768799
(43) Date of publication of application: 07.02.2001
(60) Divisional application: 05028331.6
(73) Proprietor: Dainippon Sumitomo Pharma Co., Ltd., Osaka 541-8524 (JP)
(72) Inventor: Takasu, Hideo, Nishinomiya-shi, Hyogo-ken (JP); Gotoh, Masashi, Takatsuki-shi, Osaka-fu (JP); Yamaoka, Takashi, Sanda-shi, Hyogo-ken (JP)
(74) Representative: Cresswell, Thomas Anthony

(56) References cited:
- WO-A-00/10595
- AMAGAI T: "Augmenting effect of mouse interferon-alpha/beta on cytotoxic T lymphocytes" JOURNAL OF KYOTO PREFECTURAL UNIVERSITY OF MEDICINE, vol. 97, no. 11, November 1988 (1988-11), pages 1449-1458, XP000953165
- VON HOEGEN P: "Synergistic role of type I interferons in the induction of protective cytotoxic T lymphocytes." IMMUNOLOGY LETTERS, vol. 47, no. 3, September 1995 (1995-09), pages 157-162, XP000953167
- NAGAO Y ET AL.: "Oral-mucosal administration of IFN-alpha potentiates immune response in mice." JOURNAL OF INTERFERON AND CYTOKINE RESEARCH, vol. 18, no. 9, September 1998 (1998-09), pages 661-666, XP000953170
- TÜTING T ET AL.: "Co-delivery of T helper 1-biasing cytokine genes enhances the efficacy of gene immunization of mice: studies with the model tumor antigen beta-galacosidase and the BALB/c Meth A p53 tumor-specific antigen" GENE THERAPY, vol. 6, no. 4, April 1999 (1999-04), pages 629-636, XP000953161

## Description

The present invention relates to an agent for induction of antigen-specific T cell. More specifically, the present invention relates to an agent for induction of T cell specific to antigenic peptide derived from tumor or virus.

In the elimination of cancer cells and virus-infected cells by the living body, cellular immunity, especially antigen-specific T cell [cytotoxic T cell (hereinafter also referred to as "CTL") and helper T cell] plays a central role. Antigen-specific T cell recognizes cell surface MHC molecule (in the case of human, also referred to as "HLA molecule") bound to fragment peptide from antigenic protein derived from cancer or virus, by means of T cell receptor, specifically injures cancer cells and virus-infected cells and produces various cytokines to activate immunity. Fragment peptide presented to MHC molecule is called antigenic peptide and is usually about 8 to about 20 amino acids in length. The vaccine therapy in which cancer antigenic peptide or viral antigenic peptide is administered to a living body to enhance specific T cell in the living body is thought to be useful in the treatment and prophylaxis of cancers and viral infectious diseases. In order to efficiently induce specific immunity using a vaccine, it is effective to simultaneously administer an immune-activating substance, i.e., an adjuvant, along with the principal component antigenic protein or antigenic peptide. Commonly known adjuvants include aluminum compounds and molecules derived from bacteria (*FASEB:*6, 3265, 1996; *Ann. Rev. Immunol*.:4 369, 1986). In recent years, there have been elucidated that GM-CSF and IL-12 are effective as such vaccine adjuvants. In other words, there has been reported that GM-CSF and IL-12 are administered along with antigenic peptide in the preparation form of a water-in-oil emulsion to enhance the induction of antigenic peptide-specific CTL (*J. Immunol.,* 158:3947, 1997). Furthermore, there has also been reported that GM-CSF is administered simultaneously with antigenic peptide to induce delayed-type hypersensitivity (DTH) to the same extent as with complete Freund's adjuvant, thereby being effective in inducing specific immunity *(Blood,* 88:202, 1996).

Interferons, cytokines produced by various cells, such as lymphocytes and fibroblasts, in a living body, exhibit antiviral action, anticancer action, etc., and have principal subtypes such as α, β and γ.

Interferon-α (hereinafter abbreviated as "IFN-α") is cytokine produced mainly by leukocytes and has been found as an antiviral protein produced by virus-infected cells (C.R. Seance, *Soc. Biol.,* 152:1627, 1958). IFN-α has been known to possess various biological activities, including suppressive action of tumor cell growth, enhancement for cytotoxic activity of T cells and NK cells, and enhancement for expression of MHC class I, as well as antiviral action (*Immunol. Today,* 17:369, 1996). Regarding the induction of antigen-specific immunity, there is a report that IFN-α itself is effective in inducing antigen-specific antibodies (*J. Biol. Regul. Homest. Agents,* 8:9, 1994); however, little is known about other actions.

An object of the present invention is to provide an agent for induction of antigenic peptide-specific T cells that is effective in the treatment of tumours and viral infectious diseases.

The above and other objects of the present invention will be apparent from the following description.

The present invention provides:
- use of a tumour or viral antigen peptide which binds to an MHC molecule and is recognised by CTL or a DNA capable of expressing said antigen peptide; and
- an interferon-α or a DNA capable of expressing said interferon-α in the manufacture of an agent for treatment of a tumour or a viral infectious disease by induction of CTL specific for said antigen peptide.

### BRIEF DESCRIPTION OF THE DRAWINGS

Figure 1 is graphs each showing the action of enhancing CTL induction of IFN-α in an antigenic peptide administration system by using an osmotic pump, wherein Figure 1(A) shows the cytotoxic activity for peptide-pulsed EL-4 cells, and wherein Figure 1(B) shows the cytotoxic activity for peptide-nonpulsed EL-4 cells.
Figure 2 is graphs each showing the action of enhancing CTL induction of IFN-α in an IFA dosage form, wherein Figure 2(A) shows the cytotoxic activity for peptide-pulsed EL-4 cells, and wherein Figure 2(B) shows the cytotoxic activity for peptide-nonpulsed EL-4 cells.

The present invention has been found on the basis of newly found action for enhancing induction of antigen-specific T cell possessed by interferon. When used in combination with interferon, the induction of antigen-specific T cell can be more markedly enhanced, as compared with the case of the antigenic protein, the antigenic peptide derived from the antigenic protein, or a DNA capable of expressing the antigenic protein or the antigenic peptide alone. Such an effect exhibited by the use of the agent for induction of antigen-specific T cell of the present invention can neither be anticipated nor expected from the findings of the prior art.

The phrase "antigenic proteins, antigenic peptides derived from the antigenic proteins, and DNAs capable of expressing the antigenic proteins or the antigenic peptides" as used herein refers to ones capable of inducing T cell that is specific for an antigenic peptide, and intends to encompass both of the following : ones capable of inducing antigen-specific T cell by directly forming a complex with MHC molecule (HLA molecule) on the cell surface; and ones capable of indirectly inducing antigen-specific T cell through the step comprising incorporating into cell an antigenic protein, an antigenic peptide, or a DNA capable of expressing the antigenic protein or the antigenic peptide, and thereafter binding to MHC molecule the expression product resulting from expression of the DNA or the antigenic peptide itself, or the intracellular degradation product of the expression product, the antigenic protein itself, or the like to form a complex, thereby presenting the complex to the cell surface.

The antigenic proteins include antigenic proteins derived from viruses, antigenic proteins derived from bacteria, tumor antigenic proteins, and the like. The antigenic proteins derived from viruses include antigenic proteins derived from viruses such as HIV, hepatitis C virus, hepatitis B virus, influenza virus, HPV, HTLV and EBV. The antigenic proteins derived from bacteria include antigenic proteins derived from bacteria such as *Mycobacterium tuberculosis.* A representative example of the tumor antigenic protein includes the ones listed in Table 1 in *Immunity* 10:281, 1999. Concretely, melanoma antigenic proteins, for example, include MAGE *(Science* 254:1643, 1991), gp 100 *(J. Exp. Med.* 179:1005, 1994), MART-1 *(Proc. Natl. Acad. Sci. U.S.A.* 91:3515, 1994) and tyrosinase (*J*. *Exp. Med.* 178:489, 1993); tumor antigenic proteins other than melanoma include tumor markers such as HER2/neu *(J. Exp. Med* 181:2109, 1995), CEA *(J. Natl. Cancer Inst.* 87:982, 1995) and PSA *(J. Natl. Cancer Inst.* 89:293, 1997); and SART-1 derived from squamous cell carcinoma *(J. Exp. Med.* 187, 277-288, 1998; WO 97/46676); cyclophilin B *(Proc. Natl. Acad. Sci. U.S.A.* 88:1903, 1991), and the like. These antigenic proteins may be full-length polypeptides, partial polypeptides or variants thereof, as long as they are capable of inducing antigenic peptide-specific T cell.

The antigenic peptide derived from the antigenic protein (hereinafter simply referred to as "antigenic peptide") encompasses peptides comprising a part of the antigenic protein mentioned above, each of which comprises about 8 to about 20 amino acid residues, or derivatives which are functionally equivalent thereto, or polytopes resulting from linking two or more of the peptides or the derivatives thereof. The phrase "derivatives which are functionally equivalent thereto" as used herein refers to variants resulting from substitution, deletion and/or addition (including addition of amino acids to N-terminal or C-terminal of the peptide) of one or more amino acids in the amino acid sequence of the antigenic peptide, the variants being capable of inducing antigenic peptide-specific T cell.

Concretely, the tumor antigenic peptides include the following: The tumor antigenic peptides derived from SART-1 include HLA-A26-restricted tumor antigenic peptides comprising the amino acid sequence as shown in any one of SEQ ID NO: 1 to SEQ ID NO: 3; HLA-A24-restricted tumor antigenic peptides comprising the amino acid sequence as shown in any one of SEQ ID NO: 4 to SEQ ID NO: 9; and HLA-A2-restricted tumor antigenic peptides comprising the amino acid sequence as shown in any one of SEQ ID NO: 10 to SEQ ID NO: 15. The tumor antigenic peptides derived from cyclophilin B include HLA-A24-restricted tumor antigenic peptides comprising the amino acid sequence as shown in SEQ ID NO: 16 or SEQ ID NO= 17. The tumor antigenic peptides derived from SART-3 include HLA-A24-restricted tumor antigenic peptides comprising the amino acid sequence as shown in any one of SEQ ID NO: 18 to SEQ ID NO: 24. The tumor antigenic peptides derived from ART-1 include HLA-A24-restricted tumor antigenic peptides comprising the amino acid sequence as shown in any one of SEQ ID NO: 25 to SEQ ID NO: 27. The peptides derived from yeast having activity for tumor antigenic peptide include HLA-A24-restricted peptides comprising the amino acid sequence as shown in SEQ ID NO: 28.

Examples of the antigenic peptides derived from viruses include a peptide comprising the amino acid sequence as shown in SEQ ID NO: 34, and the like.

Furthermore, derivatives that are functionally equivalent to the above-mentioned tumor antigenic peptides include peptide derivatives resulting from substitution of amino acids on the basis of the sequence regularity (motif) of antigenic peptide presented by binding to HLA antigen, if the sequence regularity has been known. Concretely, HLA-A24 binding motifs have been known to be peptides comprising 8 to 11 amino acids, of which amino acid of the 2nd position is tyrosine, phenylalanine, methionine or tryptophan, and C-terminal is phenylalanine, leucine, isoleucine, tryptophan or methionine *(J. Immunol.* 152, 3913, 1994; *Immunogenetics* 41:178, 1995; *J. Immunol.* 155:4307, 1994). In addition, as HLA-A2 binding motifs, the motifs shown in Table 1 below have been known *(Immunogenetics* 41, 178, 1995; *J*. *Immunol.* 155:4749, 1995).

**Table 1**

| Type of HLA-A2 | 2nd Amino Acid from N-terminal | C-Terminal Amino Acid |
|---|---|---|
| HLA-A0201 | L, M | V, L |
| HLA-A0204 | L | L |
| HLA-A0205 | V, L, I, M | L |
| HLA-A0206 | V, Q | V, L |
| HLA-A0207 | L | L |

| | | |
|---|---|---|
| (Length of peptide being 8 to 11 amino acids.) | | |

Therefore, examples of the tumor antigenic peptide derivatives include peptide derivatives resulting from subjecting the above antigenic peptides to amino acid substitutions acceptable for the motifs of the peptides. Concrete examples of the derivatives include tumor antigenic peptide derivatives derived from SART-1, having the amino acid sequence as shown in any one of SEQ ID NO: 29 to SEQ ID NO: 31, and tumor antigenic peptide derivatives derived from cyclophilin B, having the amino acid sequence as shown in SEQ ID NO: 32 or SEQ ID NO: 33.

The term "polytope" refers to a recombinant peptide resulting from linking a plurality of antigenic peptides (see, for example, *the Journal of Immunology* **160**, 1717, 1998). In the present invention, the polytope refers to a peptide comprising an amino acid sequence in which one or more kinds of the above-mentioned antigenic peptides or derivatives thereof are appropriately combined. The polytope can be obtained by inserting a recombinant DNA into an appropriate expression vector, the recombinant DNA being prepared by linking one or more kinds of genes encoding the above-mentioned antigenic peptides or derivatives thereof, and expressing the resulting recombinant vector in host cells.

The DNAs capable of expressing the antigenic proteins or the antigenic peptides include a DNA resulting from linking a gene encoding the above-mentioned antigenic protein or antigenic peptide to an appropriate expression vector. The gene may be genomic DNA, cDNA or chemically synthesized DNA. In addition, the gene may comprise a nucleotide sequence resulting from substitution, deletion, insertion or addition of one or more bases, as long as it encodes the antigenic protein or antigenic peptide.

The expression vectors may be any ones without particular limitation, as long as they can be expressed within antigen-presenting cells. The expression vectors include plasmid vectors and viral vectors. Preferably used plasmid vectors include commonly known vectors such as pCR3, pcDNA3.1, pRc/CMV2 (Invitrogen), pSPORT1, pSPORT2 and pSFV1 (GIBCO BRL). The viral vectors include, for example, vectors derived from DNA viruses or RNA viruses such as retrovirus, adenovirus, adeno-associated virus, herpesvirus, vaccinia virus, pox virus, poliovirus and Sindbis virus. Among these vectors, the retrovirus vector, adenovirus vector, adeno-associated virus vector and vaccinia virus vector are preferred.

The antigenic protein can be prepared by purifying by a conventional method a recombinant protein obtained by carrying out a cloning process for cDNA encoding the desired antigenic protein, a process for linking the cDNA to an expression vector, a process for introducing the vector to host cells, and a process for expressing the antigenic protein, in accordance with the basic textbooks such as *Molecular Cloning, Second Edition,* Cold Spring Harbor Laboratory Press (1989).

The antigenic peptide can be synthesized in accordance with the method used in ordinary peptide chemistry. Methods of synthesis include those described in the literatures *(Peptide Synthesis,* Interscience, New York, 1966; *The Proteins.* Vol. 2, Academic Press Inc., New York, 1976; *Pepuchido Gosei (Peptide Synthesis),* Maruzen, 1975; *Pepuchido Gosei No Kiso To Jikken (Basics and Experimentation of Peptide Synthesis),* Maruzen, 1985; *Iyakuhin No Kaihatsu, Zoku (Development of Pharmaceuticals, Sequel),* Vol. 14, *Pepuchido Gosei (Peptide Synthesis),* Hirokawa Shoten, 1991). In addition, the antigenic peptide may also be prepared by purifying by a conventional method a recombinant peptide resulting from expression of a gene encoding the antigenic peptide, in accordance with *Molecular Cloning* mentioned above.

The DNA capable of expressing the antigenic protein or the antigenic peptide can be prepared by obtaining a recombinant DNA encoding the antigenic protein or antigenic peptide, and inserting the resulting recombinant DNA into an expression vector, in accordance with the basic textbooks such as *Molecular Cloning* mentioned above.

The interferon-α and the DNA capable of expressing the interferon-α contained as another active ingredient in the agent for induction of antigen-specific T cell of the present invention enhances the induction of antigenic peptide-specific T cell.

IFN-α may be natural-occurring ones, or ones obtained by gene recombination. The natural-occurring IFN-α products include commercial products available under the trade names "Sumiferon" (Sumitomo Pharmaceuticals), "IFNα Mochida" (Mochida Pharmaceutical) and "OIF" (Otsuka Pharmaceutical). IFN-α products obtained by genetic recombination include commercial products available under the trade names "Canferon" (Takeda Chemical Industries), "Roferon" (Roche) and "Intron" (Schering-Plough). Also included are consensus interferon (Amgen) and PEG-interferon (WO 99/64016). In addition, when laboratory animals are used, in the case of a mouse, for example, there may be used commercial products such as IFN-α, murine, Recombinant, *E. coli* (Calbiochem), IFN-α, Mouse, Recombinant, CHO cells (Hycult Biotechnology), IFN-α, Mouse, Rec. (Pestka Biomedical) and interferon-α, Mouse (Paesel).

The DNAs capable of expressing the interferon-α include a DNA resulting from linking a gene encoding the interferon to an appropriate expression vector. The gene may be genomic DNA, cDNA or chemically synthesized DNA. In addition, the gene may comprise a nucleotide sequence resulting from substitution, deletion, insertion or addition of one or more bases in the nucleotide sequence, as long as the gene encodes the interferon-α. Incidentally, the nucleotide sequences of the interferons are known in the art [See *Nature* 295, 503-508 (1982); *Nature* 284, 316-320 (1980); *Nature* 290, 20-26 (1981); and *Nucleic Acids Res.* 8, 4057-4074 (1980); sequences registered at GenBank database, and the like].

The expression vectors may be any ones without particular limitation, as long as they can be expressed within a living body, and include plasmid vectors and viral vectors. Concrete examples thereof include those plasmid vectors and viral vectors exemplified above.

The DNA capable of expressing the interferon can be prepared by obtaining a recombinant DNA encoding the interferon, and inserting the resulting recombinant DNA into an expression vector, in accordance with the basic textbooks such as *Molecular Cloning* mentioned above.

Among the active ingredients contained in the agent for induction of antigen-specific T cell of the present invention, at least one is selected from the group consisting of the above-mentioned antigenic proteins, antigenic peptides and DNAs capable of expressing the antigenic proteins or the antigenic peptides. According to the purpose of treatment, two or more antigenic proteins, two or more antigenic peptides, or two or more of the DNAs may be selected.

Combinations of active ingredients contained in the agent for induction of antigen-specific T cell of the present invention include an an antigenic peptide with IFN-α, a DNA capable of expressing the antigenic peptide with IFN-α, and the like, and those combinations exemplified above in which each interferon is substituted by a DNA capable of expressing the interferon. The combination of an antigenic peptide with IFN-α is preferable, from the viewpoint that enhancement of induction of antigen-specific T cell can be carried out more specifically and effectively.

The amount of the antigenic protein or antigenic peptide in the agent for induction of antigen-specific T cell of the present invention is not subject to particular limitation, as long as antigen-specific T cell is inducible. It is preferable that the amount of the antigenic protein or antigenic peptide is usually 0.0001 to 1000 mg, preferably 0.001 to 100 mg, and more preferably 0.01 to 10 mg per administration.

The amount of the DNA capable of expressing the antigenic protein or antigenic peptide in the agent for induction of antigen-specific T cell of the present invention is not subject to particular limitation, as long as antigen-specific T cell is inducible. The amount of the DNA is preferably 0.0001 to 100 mg, more preferably 0.001 to 10 mg per administration.

The amount of the interferon in the agent for induction of antigen-specific T cell of the present invention is not subject to particular limitation, as long as antigen-specific T cell is inducible. It is preferable that the amount of the interferon is about 10 U to about 1 x 10⁸ U per administration, for IFN-α. In addition, the amount of the DNA capable of expressing the interferon is not subject to particular limitation, as long as the same level of effects is exhibited as those exhibited by the interferon in the amount exemplified above.

The ratio of the amounts of the antigenic protein, the antigenic peptide derived from the antigenic protein, the DNA capable of expressing the antigenic protein or the antigenic peptide, the interferon, and the DNA capable of expressing the interferon in the agent for induction of antigen-specific T cell of the present invention is not subject to particular limitation. Each active ingredient may be contained in the agent such that each active ingredient can be administered in the amount mentioned above, per administration of the agent.

The agent for induction of antigen-specific T cell of the present invention may further comprise an immunopotentiator. The term "immunopotentiator" as used herein refers to a substance possessing a so-called adjuvant activity which enhances cellular immunity induction specific to an antigen, when administered together with the antigen. In the present invention, the immunopotentiator may also include cytokines other than the interferons. The immunopotentiators include, for example, the adjuvants described in the publication (*Clin. Microbiol. Rev.* 7:277, 1994), concretely aluminum compounds, *C. parvum* derived from bacteria, BCG-CWS, lipopolysaccharide (LPS) or muramyl dipeptide (MDP), saponin derived from plants, and the like. The cytokines other than the interferon include GM-CSF, IL-12, IL-2, and the like.

It is preferable that the agent for induction of antigen-specific T cell of the present invention is in a preparation form so as to exhibit the desired pharmacological effect. Preparation forms suitable for this purpose include water-in-oil (w/o) emulsion preparations, oil-in-water (o/w) emulsion preparations, water-in-oil-in-water (w/o/w) emulsion preparations, liposome preparations, microsphere preparations, microcapsule preparations, solid injection preparations, and liquid preparations.

The water-in-oil (w/o) emulsion preparation takes the form in which active ingredients are dispersed in a water dispersion phase.

The oil-in-water (o/w) emulsion preparation takes the form in which active ingredients are dispersed in a water dispersion medium.

The water-in-oil-in-water (w/o/w) emulsion preparation takes the form in which active ingredients are dispersed in the innermost water dispersion phase.

The above emulsions can be prepared by referring to, for example, Japanese Patent Laid-Open Nos. Hei 8-985, Hei 9-122476, and the like.

The liposome preparation comprises fine particles taking the form in which active ingredients are enveloped in an aqueous phase or a membrane by means of a liposome having a lipid bilayer structure. Principal lipids for making liposome include phosphatidylcholine, sphingomyelin, and the like, to which lipids dicetyl phosphate, phosphatidic acid, phosphatidylserine, or the like, is added to charge and stabilize the liposome. Methods of making the liposome include ultrasonication method, ethanol injection method, ether injection method, reverse-phase evaporation method and French press extraction method.

The microsphere preparation comprises a homogenous polymeric matrix, and it is composed of fine particles taking the form in which active ingredients are dispersed in the matrix. Materials for the matrix include biodegradable polymers such as albumin, gelatin, chitin, chitosan, starch, polylactic acid and polyalkylcyanoacrylates. Methods of making the microsphere preparation may be those in accordance with the commonly known methods without particular limitation.

The microcapsule preparation is composed of fine particles taking the form in which active ingredients as the core substances are coated with a coating substance. The materials used for the coating substance include membrane-forming polymers such as carboxymethyl cellulose, cellulose acetate phthalate, ethyl cellulose, gelatin, gelatin-gum arabic, nitrocellulose, polyvinyl alcohol and hydroxypropyl cellulose. Methods of making the microcapsule preparation include coacervation method and interfacial polymerization method.

The solid injection preparation takes a preparation form in which active ingredients are sealed in a substrate made of collagen, silicon or the like, and solidified. Methods of making the solid injection preparation include a method described in publications such as *Pharm. Tech. Japan* 7, 402-409 (1991).

The liquid preparation takes the form in which active ingredients are mixed with pharmaceutically acceptable solvents, carriers, and the like. The pharmaceutical acceptable solvents include water, glucose solutions, physiological saline, and the like. Furthermore, pharmaceutical acceptable auxiliaries, for example, pH regulators or buffers, tension regulators, wetting agents, and the like may be contained.

The agent for induction of antigen-specific T cell of the present invention described above may be previously formed into a preparation or freshly prepared when administered to a patient. In other words, an antigenic protein, an antigenic peptide, a DNA capable of expressing the antigenic protein or the antigenic peptide, an interferon, a DNA capable of expressing the interferon, an immunopotentiator, and the like, which are active ingredients of the agent for induction of antigen-specific T cell of the present invention, and the preparation form emulsion and the like, may be previously mixed and formed into a preparation, or freshly prepared when administered to a patient.

The induction ability of antigen-specific T cell can be evaluated as described below for the agent for induction of antigen-specific T cell of the present invention prepared as described above.

The agent for induction of antigen-specific T cell of the present invention is one or more times administered to a laboratory animal. After 1 week from final administration, the spleen is extirpated, to prepare splenic lymphocytes. Splenocytes from an unsensitized mouse are also prepared, and pulsed for several hours with an antigenic peptide. Thereafter, the pulsed splenocytes are irradiated with an X-ray at about 2000 to about 5000 rad, and these cells are used as the antigen-presenting cells. By adding the antigen-presenting cells to lymphocytes from the immunized mouse, re-stimulation with the antigenic peptide is carried out in a culture system. The same stimulation is carried out plural times at a frequency of once a week as needed. After 1 week from final stimulation, lymphocytes are collected. The induction ability of antigen-specific T cell can be evaluated, for instance, by quantifying various cytokines (for example, IFN-γ) produced in response to antigenic peptide-specific T cell induced in the lymphocytes, with antigenic peptide-pulsed cells, antigen-positive cells, and the like as target cells, or by determining the cytotoxic activity of antigenic peptide-specific T cell on ⁵¹Cr-labeled target cells by the ⁵¹Cr release determination method (*J. Immunol*. 139:2888, 1987). In the case of human, the induction ability of antigenic peptide-specific T cell can be evaluated in a similar method using peripheral blood monocytes (PBMC) separated from peripheral blood by the Ficoll method or the like in place of the splenic lymphocytes from a laboratory animal.

When the agent for induction of antigen-specific T cell of the present invention is administered to an antigen-positive patient, the antigenic peptide is presented at high density to the HLA antigen of antigen-presenting cells, so that the presented HLA antigen complex-specific T cell grows to destroy target cells (antigen-peptide-positive cells) or to produce various cytokines, whereby immunity can be activated. Preferably, the agent for induction of antigen-specific T cell of the present invention is used for treatment or prophylaxis of a tumor or viral infectious disease. When used for treatment or prophylaxis of a tumor, the agent for induction of antigen-specific T cell of the present invention comprising a tumor-specific tumor antigenic peptide as an active ingredient is administered to a patient, so that the cellular immunity specific to cancer cells is enhanced, whereby the tumor can be treated, or growth and metastasis of the tumor can be prevented. Furthermore, the agent for induction of antigen-specific T cell of the present invention may be used in combination with conventional chemotherapy or radiotherapy, whereby achieving a greater therapeutic effect.

When used for treatment or prophylaxis of a viral infectious disease, the agent for induction of antigen-specific T cell of the present invention comprising an antigenic peptide derived from virus as an active ingredient is administered to a patient, so that cellular immunity specific to virus-infected cells is enhanced, whereby the viral infectious disease can be treated or prevented.

Methods of administration include subcutaneous injection, persistent subcutaneous injection, intracutaneous injection, intravenous injection, intraarterial injection, intramuscular injection, topical injection and intraperitoneal administration. Continuous gradual administration using an osmotic pump or the like and implantation of a. sustained-release preparation (for example, mini-pellet preparation) can be carried out. The frequency of administration is not subject to particular limitation, and it is preferably once per several days to several months.

Furthermore, the present invention also relates to an enhancing agent for induction of antigen-specific T cell comprising at least one of interferons and DNAs capable of expressing the interferons as an active ingredient. As described above, the interferon, which is an active ingredient of the agent for induction of antigen-specific T cell of the present invention, exhibits an effect for enhancing the induction of antigen-specific T cell. Therefore, in addition to its use as a component of the agent for induction, the interferon can also be used alone as an enhancing agent for induction for increasing the induction activity for antigen-specific T cell. The enhancing agent for induction of the present invention is effectively used, for example, in cases where T cell induction activity .with vaccine is insufficient. It is especially preferable to use interferon-α.

The amount of the interferon in the enhancing agent for induction of the present invention is not subject to particular limitation, as long as antigen-specific T cell induction can be enhanced. The amount of the interferon is about 10 U to about 1 x 10⁸ U per administration, for IFN-α. In addition, the amount of the DNA capable of expressing the interferon is not subject to particular limitation, as long as the same level of effects is exhibited as those exhibited by the interferon in the amount exemplified above.

The method of preparing the enhancing agent for induction of the present invention and the method of administering the enhancing agent for induction are the same as those for the above-mentioned agent for induction of antigen-specific T cell.

### EXAMPLES

### Example 1

### Action of Enhancing CTL Induction of IFN-α in Antigenic Peptide Administration System by Using Osmotic Pump

CTL induction was examined in a system in which an osmotic pump (Alza, Model "1003D") was charged with the drug and subcutaneously implanted in a mouse to release the drug over a 3-day period. The following five experimental groups were set:
Group 1: 100 µg of peptide Flu₃₆₆₋₃₇₄ was administered using the osmotic pump.
Group 2: 10⁵ units (hereinafter abbreviated U) of IFN-α was administered using the osmotic pump.
Group 3: 100 µg of peptide Flu₃₆₆₋₃₇₄ and 10⁵ U of IFN-α were administered using the osmotic pump.
Group 4: 100 µg of peptide Flu₃₆₆₋₃₇₄ as mixed with incomplete Freund's adjuvant was administered.
Group 5: Not treated (without drug administration).

The amino acid sequence of peptide Flu₃₆₆₋₃₇₄, which is an H-2D^{b} restrictive antigen peptide derived from influenza virus, is Ala-Ser-Asn-Glu-Ser-Met-Glu-Thr-Met (SEQ ID NO: 34), and the peptide was synthesized by the Fmoc method.

Incomplete Freund's adjuvant (hereinafter abbreviated "IFA") was purchased from Wako Pure Chemical Industries. An emulsion was freshly prepared before use by connecting two glass syringes and mixing an equal volume of IFA and a peptide solution (2 mg/ml).

Mouse IFN-α was prepared by infecting the mouse cell line EAT cells (derived from Ehrlich ascites carcinoma, ATCC Stock Number CCL-77), previously treated with sodium butyrate, with the Newcastle disease viruses (hereinafter abbreviated "NDV"), treating the infected cells with theophylline to produce IFN-α, and purifying the resulting IFN-α using a CPG column and anti-IFN-α antibody column. IFN-α titer was determined by the measurement method with inhibition of viral cytopathic effect (CPE) by IFN-α as an index with reference to the method described in a book (M.J. Clemens et al., edts., *Lymphokines and Interferons: a practical approach,* p*.* 6, IRL Press, Oxford, 1987).

For Groups 1 to 3, the drug-filled osmotic pump was subcutaneously implanted at the tail base of C57BL/6 mouse (Charles River Japan). For Group 4, 0.1 ml of an IFA emulsion was subcutaneously administered to the tail base of C57BL/6 mouse. For each group, three mice were used.

After 7 days from the drug administration, the spleen was extirpated from the mice in each group and subjected to hemolytic treatment to prepare splenocytes. The splenocytes were sown to 10 wells of a 24-well plate at 5 x 10⁶ cells/well (1.7 ml). Splenocytes for antigen-presenting cells were prepared from non-treated mouse spleen, and peptide-pulsed by adding 90 µg/ml of the peptide Flus₃₆₆₋₃₇₄, and culturing the mixture at 37°C for 1 hour. Thereafter, the peptide-pulsed splenocytes were irradiated with an X-ray (2000 rad). These resulting antigen-presenting cells were added to the above 24-well plates sown with the mouse splenocytes for Groups 1 to 5 at 5 x 10⁶ cells/well (0.1 ml), to conduct re-stimulation with the peptide *in vitro.* In the culture, there was employed a culture medium of RPMI 1640 supplemented with 10% FCS, 10 U/ml mouse IL-2 (BECTON DICKINSON), 10 mM HEPES, 20 mM L-glutamine, 1 mM sodium pyruvate, 1 mM MEM non-essential amino acids (GIBCO BRL), 1% MEM vitamins (GIBCO BRL) and 55 µM 2-mercaptoethanol.

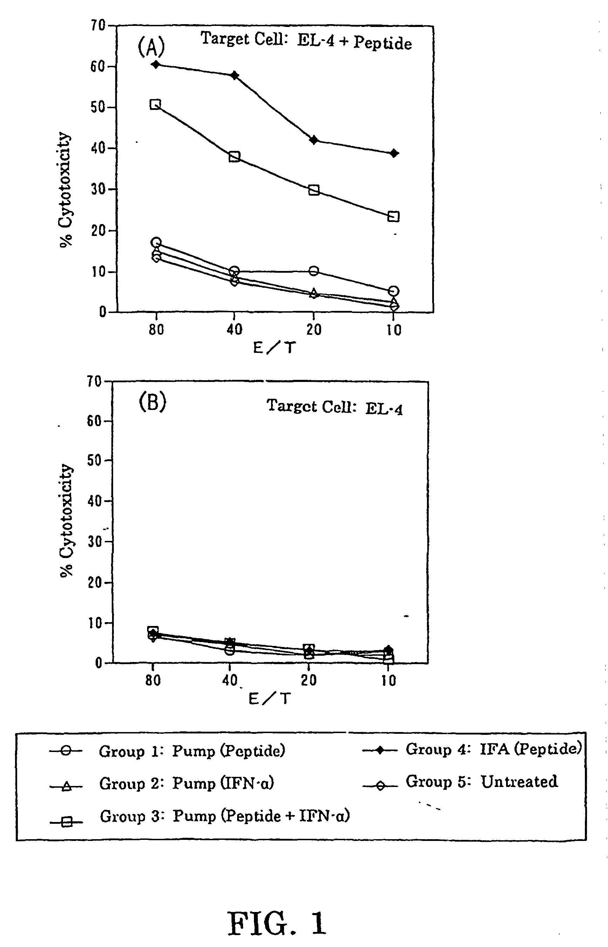

After 5 days of culture, the splenocytes were collected from the plates and subjected to the ⁵¹Cr release determination method in accordance with the method described in T. Nishimura et al., *J. lmmunol.,* 139, 2888 (1987) to determine the peptide-specific cytotoxic activity. The target cells used for determination of the peptade-specific cytotoxic activity were EL-4 cells (derived from lymphoma, ATCC Stock Number TIB-39) labeled with ⁵¹Cr and pulsed with the peptide Flu_{366·374}. The target cells used for determination of the non-specific cytotoxic activity were EL-4 cells labeled with ⁵¹Cr. The results are shown in Fig. 1(A) and Fig. 1(B). As shown in Fig. 1(B), the cytotoxic activity for peptide-nonpulsed EL-4 cells was as low as 10% or less in all groups; in the present example, non-specific killer cells were not induced. Fig. 1(A) shows the cytotoxic activity for peptide-pulsed EL-4 cells. As shown in Fig. 1(A), no peptide-specific CTL was induced when the peptide or IFN-α was administered alone using the osmotic pump (Groups 1 and 2), as the case of "not treated" (Group 5). When IFN-α was administered simultaneously with the peptide by using the osmotic pump, however, the peptide-specific cytotoxic activity was detected as the case of IFA administration. Therefore, it was clarified that CTL was induced. It has been clarified from the results that IFN-α possesses the action of enhancing CTL induction.

### Example 2

### Action of Enhancing CTL Induction of IFN-α in IFA Preparation Form

The action of IFN-α in a system for inducing specific CTL by administering an antigenic peptide in an IFA emulsion preparation form was evaluated.
Group 1: 100 µg of peptide Flu₃₆₆₋₃₇₄ was administered in the IFA preparation form.
Group 2: 100 µg of peptide Flu₃₆₆₋₃₇₄ and 10⁵ U of IFN-α were administered in the IFA preparation form.
Group 3: The IFA preparation form (vehicle) was administered.

The peptide Flu₃₆₆₋₃₇₄, IFA and IFN-α used were of the same lots as those used in Example 1. An emulsion was freshly prepared before use by connecting two glass syringes and mixing an equal volume of the drug solution prepared with PBS and IFA. This emulsion in an amount of 0.1 ml was subcutaneously administered to the tail base of C57BL/6 mouse. For each group, three mice were used. After 7 days from the drug administration, splenocytes were prepared, and thereafter re-stimulation was carried out with the peptide in the same manner as in Example 1. After 5 days of culture, the cytotoxic activity was determined by the ⁵¹Cr release method.

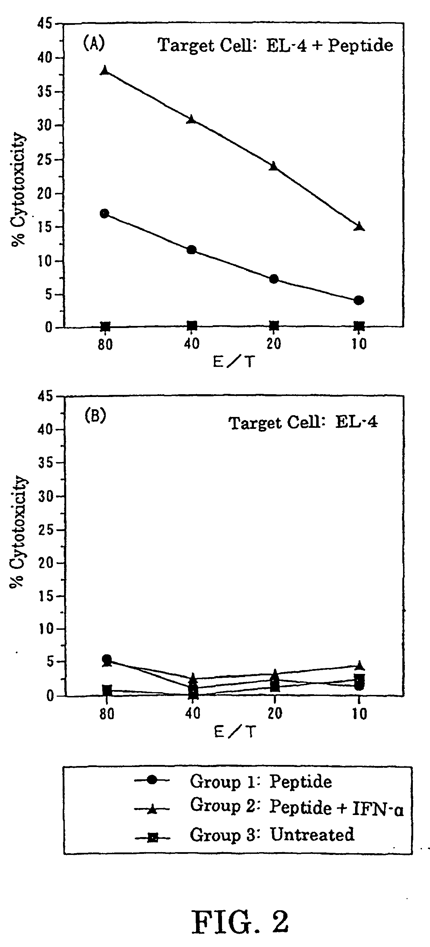

The results are shown in Fig. 2(A) and Fig. 2(B). As shown in Fig. 2(B), the cytotoxic activity for peptide-nonpulsed EL-4 cells was as low as 10% or less in all groups; in the present example, non-specific killer cells were not induced. Fig. 2(A) shows the cytotoxic activity for peptide-pulsed EL-4 cells. As shown in Fig. 2(A), more potent cytotoxic activity was induced in the group subjected to administration of the peptide and IFN-α (Group 2) than in the group subjected to administration of the peptide alone (Group 1), in the IFA form. It was clarified from the results that IFN-α exhibits the action of enhancing induction of peptide-specific CTL induction even in the IFA preparation form.

In the agent for induction of antigenic-specific T cell of the present invention, since interferons such as IFN-α, which are active ingredients, enhance the action of antigenic peptide-specific T cell induction, the agent is useful as a vaccine purposed for induction of antigenic peptide-specific cellular immunity.

### FREE TEXT FOR SEQUENCE LISTING

SEQ ID NO: 29 is a designed peptide based on the motif for antigenic peptide from SART-1, to which HLA antigen binds.
SEQ ID NO: 30 is a designed peptide based on the motif for antigenic peptide from SART-1, to which HLA antigen binds.
SEQ ID NO: 31 is a designed peptide based on the motif for antigenic peptide from SART-1, to which HLA antigen binds.
SEQ ID NO: 32 is a designed peptide based on the motif for antigenic peptide from cyclophilin B, to which HLA antigen binds.
SEQ ID NO: 33 is a designed peptide based on the motif for antigenic peptide from cyclophilin B, to which HLA antigen binds.

## Claims

1. Use of
a tumour or viral antigen peptide which binds to an MHC molecule and is recognised by CTL or a DNA capable of expressing said antigen peptide; and
an interferon-α or a DNA capable of expressing said interferon-α
in the manufacture of an agent for treatment of a tumour or a viral infectious disease by induction of CTL specific for said antigen peptide,

2. Use according to claim 1, wherein an antigen peptide as defined in claim 1, and either interferon-α or a DNA capable of expressing interferon-α are used in the manufacture of said agent.

3. Use according to any one of the preceding claims wherein the agent further comprises an immunopotentiator.

4. Use according to any one of the preceding claims, wherein the agent is in a preparation form selected from water-in-oil (w/o) emulsion preparations, oil-in-water (o/w) emulsion preparations, water-in-oil-in-water (w/o/w) emulsion preparations, liposome preparations, microsphere preparations, microcapsule preparations, solid injection preparations and liquid preparations,

## Revendications

1. Utilisation
d'un peptide antigénique tumoral ou viral qui se lie à une molécule du CMH et qui est reconnu par des CTL ou d'un ADN capable d'exprimer ledit polypeptide antigénique ;
et
d'un interféron-α ou d'un ADN capable d'exprimer ledit interféron-α dans la fabrication d'un agent pour le traitement d'une tumeur ou d'une maladie infectieuse virale par induction de CTL spécifiques dudit peptide antigénique.

2. Utilisation selon la revendication 1 où un peptide antigénique tel qu'il est défini dans la revendication 1 et un interféron-α ou un ADN capable d'exprimer un interféron-α sont utilisés dans la fabrication dudit agent.

3. Utilisabon selon l'une quelconque des revendications précédentes où l'agent comprend en outre un immunopotentiateur.

4. Utilisation selon l'une quelconque des revendications précédentes où l'agent est dans une forme de préparation choisie parmi les préparations de type émulsions eau dans huile (e/h), les préparations de type émulsions huile dans eau (h/e), les préparations de type émulsions eau dans huile dans eau (e/h/e), les préparations de type liposomes, les préparations de type microsphères, les préparations de type microcapsules, les préparations pour injection solides et les préparations liquides,

## Patentansprüche

1. Verwendung
eines Tumor- oder viralen Antigenpeptids, das an ein MHC-Molekül bindet und von CTL erkannt wird, oder einer DNA, die in der Lage ist, das Antigenpeptid zu exprimieren; und
eines Interferons-α oder einer DNA, die in der Lage ist, das Interferon-α zu exprimieren
für die Herstellung eines Mittels zur Behandlung eines Tumors oder einer viralen infektiösen Krankheit durch Induzierung von CTL, die spezifisch für das Antigenpeptid sind.

2. Verwendung nach Anspruch 1, wobei ein Antigenpeptid wie in Anspruch 1 definiert und entweder Interferon-α oder eine DNA, die in der Lage ist, Interferon-α zu exprimieren, für die Herstellung des Mittels verwendet werden.

3. Verwendung nach einem der vorangegangenen Ansprüche, wobei das Mittel des weiteren einen Immunpotentiator umfasst.

4. Verwendung nach einem der vorangegangenen Ansprüche, wobei das Mittel in einer Zubereitungsform ist, die ausgewählt ist aus Wasser in-Öl (w/o)-Emulsionszubereitungen, Öl-in-Wasser (o/w)-Emulsionszubereitungen, Wasser-in-Öl-in-Wasser (w/o/w)-Emulsionszubereitungen, Liposomen-Zubereitungen, Mikrosphären-Zubereitungen, Mikrokapsel-Zubereitungen, festen Injektionszubereitungen und flüssigen Zubereitungen.


### SEQUENCE LISTING

<110> Sumitomo Pharmaceuticals Co., Ltd.
<120> Agent for Induction of Antigen-Specific T Cell
<150> JP 11-207687
   <151> 1999-07-22
<160> 34
<210> 1
   <211> 9
   <212> PRT
   <213> Homo sapiens
<400> 1
   Lys Gly Ser Gly Lys Met Lys Thr Glu
   5
<210> 2
   <211> 9
   <212 > PRT
   <213> Homo sapiens
<400> 2
   Lys Leu Asp Glu Glu Ala Leu Leu Lys
   5
<210> 3
   <211>9
   <212> PRT
   <213> Homo sapiens
<400> 3
   Val Leu Ser Gly Ser Gly Lys Ser Met
   5
<210> 4
   <211> 9
   <212> PRT
   <213> Homo sapiens
<400> 4
   Glu Tyr Arg Gly Phe Thr Gln Asp Phe
   5
<210> 5
   <211> 9
   <212> PRT
   <213> Homo sapiens
<400> 5
   Ala Phe Arg Gln Leu Ser His Arg Phe
   5
<210> 6
   <211> 11
   <212> PRT
   <213> Homo sapiens
<400> 6
   Lys Met Lys Thr Glu Arg Arg Met Lys Lys Leu
   5 10
<210> 7
   <211> 10
   <212> PRT
   <213> Homo sapiens
<400> 7
   Arg Met Lys Lys Leu Asp Glu Glu Ala Leu
   5 10
<210> 8
   <211> 11
   <212> PRT
   <213> Homo sapiens
<400> 8
   Arg Met Lys Lys Leu Asp Glu Glu Ala Leu Leu
   5 10
<210> 9
   <211> 9
   <212> PRT
   <213> Homo sapiens
<400> 9
   Lys Met Ser Ser Ser Asp Thr Pro Leu
   5
<210> 10
   <211> 9
   <212> PRT
   <213> Homo sapiens
<400> 10
   Leu Leu Leu Cys Gln Asn Lys Gly Leu
   5
<210> 11
   <211> 10
   <212> PRT
   <213> Homo sapiens
<400> 11
   Leu Leu Leu Cys Gln Asn Lys Gly Leu Leu
   5 10
<210> 12
   <211> 9
   <212> PRT
   <213> Homo sapiens
<400> 12
   Leu Leu Glu Thr Thr Val Gln Lys Val
   5
<210> 13
   <211> 9
   <212> PRT
   <213> Homo sapiens
<400> 13
   Arg Val Lys Ala Pro Asn Lys Ser Leu
   5
<210> 14
   <211> 9
   <212>PRT
   <213> Homo sapiens
<400> 14
   Tyr Val Asp Glu Thr Gly Arg Lys Leu
   5
<210> 15
   <211> 9
   <212> PRT
   <213> Homo sapiens
<400> 15
   Ala Gln Lys Thr Pro Tyr Ile Val Leu
   5
<210> 16
   <211> 9
   <212> PRT
   <213> Homo sapiens
<400> 16
   Lys Phe His Arg Val Ile Lys Asp Phe
   1 5
<210> 17
   <211> 9
   <212> PRT
   <213> Homo sapiens
<400> 17
   Asp Phe Met Ile Gln Gly Gly Asp Phe
   1 5
<210> 18
   <211> 10
   <212> PRT
   <213> Homo sapiens
<400> 18
   Val Tyr Asp Tyr Asn Cys His Val Asp Leu
   5 10
<210> 19
   <211> 10
   <212> PRT
   <213> Homo sapiens
<400> 19
   Leu Phe Glu Lys Ala Val Lys Asp Tyr Ile
   5 10
<210> 20
   <211> 9
   <212> PRT
   <213> Homo sapiens
<400> 20
   Asn Tyr Asn Lys Ala Leu Gln Gln Leu
   5
<210> 21
   <211> 9
   <212> PRT
   <213> Homo sapiens
<400> 21
   Ala Tyr Ile Asp Phe Glu Met Lys Ile
   5
<210> 22
   <211> 10
   <212> PRT
   <213> Homo sapiens
<400> 22
   Asp Tyr Val Glu Ile Trp Gln Ala Tyr Leu
   5 10
<210> 23
   <211>9
   <212> PRT
   <213> Homo sapiens
<400> 23
   Asp Tyr Leu Arg Arg Arg Val Asp Phe
   5
<210> 24
   <211> 9
   <212> PRT
   <213> Homo sapiens
<400> 24
   Ala Phe Thr Arg Ala Leu Glu Tyr Leu
   5
<210> 25
   <211> 10
   <212> PRT
   <213> Homo sapiens
<400> 25
   Gly Phe Asp Cys Ala Asn Glu Ser Val Leu
   5 10
<210> 26
   <211> 9
   <212> PRT
   <213> Homo sapiens
<400> 26
   Glu Tyr Cys Leu Lys Phe Thr Lys Leu
   5
<210> 27
   <211>8
   <212> PRT
   <213> Homo sapiens
<400> 27
   Leu Tyr Gln Ala Val Ala Thr Ile
   5
<210> 28
   <211>9
   <212 > PRT
   <213> Saccharomyces cerevisiae
<400> 28
   Glu Tyr Arg Gly Phe Thr Pro Met Phe
   5
<210> 29
   <211> 9
   <212> PRT
   <213> Artificial Sequence
   <220>
   <221> VARIANT
   <223> Designed peptide based on the motif for antigenic peptide from SART-1, to which HLA antigen binds.
<400> 29
   Glu Tyr Arg Gly Phe Thr Gln Asp Ile
   5
<210> 30
   <211>9
   <212> PRT
   <213> Artificial Sequence
   <220>
   <221> VARIANT
   <223> Designed peptide based on the motif for antigenic peptide from SART-1, to which HLA antigen binds.
<400> 30
   Glu Tyr Arg Gly Phe Thr Gln Asp Leu
   5
<210> 31
   <211> 9
   <212> PRT
   <213> Artificial Sequence
   <220>
   <221> VARIANT
   <223> Designed peptide based on the motif for antigenic peptide from SART-1, to which HLAantigen binds.
<400> 31
   Glu Tyr Arg Gly Phe Thr Gln Asp Trp
   5
<210> 32
   <211> 9
   <212> PRT
   <213> Artificial Sequence
   <220>
   <221> VARIANT
   <223> Designed peptide based on the motif for antigenic peptide.from cyclophilin B, to which HLA antigen binds.
<400> 32
   Lys Tyr His Arg Val Ile Lys Asp Phe
   1 5
<210> 33
   <211> 9
   <212> PRT
   <213> Artificial Sequence
   <220>
   <221> VARIANT
   <223> Designed peptide based on the motif for antigenic peptide from cyclophilin B, to which HLA antigen binds.
<400> 33
   Asp Tyr Met Ile Gln Gly Gly Asp Phe
   1 5
<210> 34
   <211>9
   <212> PRT
   <213> Influenza virus
<400> 34
   Ala Ser Asn Glu Ser Met Glu Thr Met
   5